# EUROPÄISCHE PATENTSCHRIFT

(11) **EP 3 287 915 B1**
(45) Veröffentlichungstag und Bekanntmachung des Hinweises auf die Patenterteilung: **08.11.2023**
(21) Anmeldenummer: 16185605.9
(22) Anmeldetag: 25.08.2016
(51) Int. Cl.: G16H 40/67, G16H 40/63, G16H 30/20, H04L 9/40

(54) **ERZEUGUNG EINER FAVORITENMENGE UMFASSEND MEHRERE PROTOKOLLE ZUR ANSTEUERUNG EINES MEDIZINISCHEN BILDGEBUNGSGERÄTES**
GENERATION OF MULTIPLE PROTOCOLS COMPRISING AN AMOUNT OF FAVOURITES FOR CONTROLLING A MEDICAL IMAGING DEVICE
GENERATION D'UNE QUANTITE DE FAVORIS COMPRENANT PLUSIEURS PROTOCOLES DESTINES A COMMANDER UN APPAREIL D'IMAGERIE

(43) Veröffentlichungstag der Anmeldung: 28.02.2018
(73) Patentinhaber: Siemens Healthcare GmbH, 91052 Erlangen (DE)
(72) Erfinder: KEIL, Miriam, 91056 Erlangen-Dechsendorf (DE)

(56) Entgegenhaltungen:
- DE-A1-102014 219 405
- US-A1- 2005 267 348

## Beschreibung

Die Erfindung betrifft ein Verfahren, ein medizinisches Bildgebungsgerät, ein Computerprogrammprodukt sowie einen computerlesbaren Datenträger zu einer Erzeugung einer Favoritenmenge umfassend mehrere Protokolle, wobei jedes Protokoll der mehreren Protokolle Steuerparameter zur Ansteuerung des medizinischen Bildgebungsgerätes umfasst. Die Erfindung ist in dem beigefügten Satz von Ansprüchen dargelegt.

Medizinische Bildgebungsgeräte weisen meist mehrere Bauteile auf, deren Ansteuerung typischerweise mittels einer Software, die Algorithmen umfasst, ausgeführt wird. Medizinische Bildgebungsgeräte können so beispielsweise Bilddaten von Patienten oder anderen Untersuchungsobjekten erzeugen. Insbesondere können basierend auf den Bilddaten klinische Diagnosen erstellt werden und gegebenenfalls Krankheiten diagnostiziert werden. Abhängig von der klinischen Fragestellung können die Art des zu verwendenden medizinischen Bildgebungsgerätes und typischerweise Steuerparameter für die Ansteuerung des medizinischen Bildgebungsgerätes gewählt werden. Diese, meist mehreren Steuerparameter werden typischerweise als Protokoll bezeichnet. Für die Untersuchung eines Patienten kann es erforderlich sein, dass das medizinische Bildgebungsgerät mit mehreren, voneinander verschiedenen Protokollen angesteuert wird.

Typischerweise kann für die Ansteuerung eines medizinischen Bildgebungsgerätes ein Protokoll aus mehreren Protokollen ausgewählt und gegebenenfalls an die auszuführende Untersuchung oder an den Patienten individuell angepasst werden. Die mehreren Protokolle, welche zur Auswahl stehen, hängen typischerweise von dem medizinischen Bildgebungsgerät und/oder von einem vom medizinischen Bildgebungsgerät umfassten Bauteil und/oder der installierten Software ab. Zusätzlich können Wünsche eines Benutzers des medizinischen Bildgebungsgerätes bei den zur Auswahl stehenden Protokollen berücksichtigt sein. Die zur Auswahl stehenden, mehreren Protokolle können als Favoritenmenge bezeichnet werden.

Die Favoritenmenge wird typischerweise bei Installation des medizinischen Bildgebungsgerätes bestimmt. Bei Änderung eines vom medizinischen Bildgebungsgerät umfassten Bauteils und/oder bei Installation einer Software kann eine Anpassung der Favoritenmenge erforderlich und/oder vorteilhaft sein. Die Bestimmung und/oder die Anpassung der Favoritenmenge wird typischerweise von einer geschulten Fachkraft ausgeführt.

US 2005/0267348 A1 beschreibt ein Verfahren zur Bildgebung eines Patienten, welches eine automatische Protokollauswahl vorsieht. DE 10 2014 219405 A1 beschreibt ein Verfahren zur Optimierung einer Untersuchung mittels eines medizinischen Bildgebungsgerätes, umfassend eine Auswahl eines Untersuchungsprotokolls.

Der Erfindung liegt die Aufgabe zugrunde, ein Verfahren zu einer Erzeugung einer Favoritenmenge umfassend mehrere Protokolle zur Ansteuerung eines medizinischen Bildgebungsgerätes anzugeben, welches eine besonders individuelle und einfache Erzeugung der Favoritenmenge gemäß den Bedürfnissen eines Benutzers des medizinischen Bildgebungsgerätes ermöglicht. Die Aufgabe wird durch die Merkmale der unabhängigen Ansprüche gelöst. Vorteilhafte Ausgestaltungen sind in den Unteransprüchen beschrieben.

Das erfindungsgemäße Verfahren zu einer Erzeugung einer Favoritenmenge umfassend mehrere Protokolle, wobei jedes Protokoll der mehreren Protokolle Steuerparameter zur Ansteuerung eines medizinischen Bildgebungsgerätes umfasst, umfasst die folgenden Verfahrensschritte:
- Ermittlung eines Systemparameters des medizinischen Bildgebungsgerätes,
- Vorgabe einer Anforderung betreffend eine Aufnahme von medizinischen Bilddaten unabhängig von einem individuellen Untersuchungsobjekt durch einen Benutzer des medizinischen Bildgebungsgerätes,
- Bestimmung der Favoritenmenge durch Erstellung zumindest eines Protokolls unter Berücksichtigung des Systemparameters und der Anforderung mittels einer Bestimmungseinheit,
- Bereitstellung der Favoritenmenge über einen Ausgang,
wobei bei Nichterfüllung von zumindest einer Anforderung bei Bestimmung der Favoritenmenge dem Benutzer ein Protokoll zur Auswahl vorgeschlagen wird, welches die zumindest eine Anforderung weitgehend erfüllt.

Das erfindungsgemäße Verfahren wird durch ein medizinisches Bildgebungsgerät mit einer Steuerungseinheit, die eine Bestimmungseinheit umfasst, ausgeführt.

In der medizinischen Bildgebung werden Bilddaten erstellt, die die Anatomie oder innere Struktur von einem Untersuchungsobjekt in Form von Schnittbildern oder Projektionen darstellen. Ein Untersuchungsobjekt kann beispielsweise ein Patient, eine Trainingsperson oder ein Phantom sein. Ein medizinisches Bildgebungsgerät ist ein System, das zu einer Ausführung einer medizinischen Bildgebung ausgebildet ist. Beispiele für medizinische Bildgebungsgeräte sind Röntgengeräte, Ultraschallgeräte, Computertomographen und Magnetresonanzgeräte.

Zur Verwendung eines medizinischen Bildgebungsgerätes für die medizinische Bildgebung wird das medizinische Bildgebungsgerät und/oder vom medizinischen Bildgebungsgerät umfasste Bauteile typischerweise angesteuert, wobei abhängig vom medizinischen Bildgebungsgerät verschiedene Steuerparameter gewählt werden können. Die gewählten Steuerparameter können von dem medizinischen Bildgebungsgerät, vom Untersuchungsobjekt und/oder von der klinischen Fragestellung abhängen. Steuerparameter können physikalische Größen wie beispielsweise eine an ein Bauteil, beispielsweise an eine Hochfrequenz-Spule oder an eine Röntgenröhre, anzulegende Spannung sein. Ist das medizinische Bildgebungsgerät beispielsweise ein Magnetresonanzgerät, so kann ein Steuerparameter eine Dauer zwischen zwei auszuspielenden Hochfrequenzpulsen und/oder Magnetfeldgradienten sein. Steuerparameter können die aufzunehmenden Bilddaten charakterisieren, indem sie beispielsweise eine Auflösung vorgeben.

Typischerweise werden Steuerparameter abhängig von dem Untersuchungsobjekt und/oder einer klinischen Fragestellung, insbesondere abhängig von der Ursache der Untersuchung mittels des medizinischen Bildgebungsgeräts, gewählt. Ein Set von Steuerparametern, basierend auf welchem Set das medizinische Bildgebungsgerät angesteuert werden kann und Bilddaten erzeugt werden können, kann als Protokoll bezeichnet werden. Die mit den Steuerparametern eines Protokolls erzeugten Bilddaten weisen vorzugsweise einen einheitlichen Kontrast auf und stellen vorzugsweise einen Teilbereich des Untersuchungsobjektes dar. Insbesondere bei Untersuchungen mit Magnetresonanzgeräten wird bei einer Untersuchung ein Teilbereich des Untersuchungsobjektes in zumindest einem weiteren Kontrast dargestellt. Hierfür kann ein weiteres Protokoll umfassend andere Steuerparameter verwendet werden. Für eine Untersuchung eines Untersuchungsobjektes kann also die Ansteuerung des medizinischen Bildgebungsgerätes mittels mehrerer Protokolle erfolgen.

Bei der Ansteuerung des medizinischen Bildgebungsgerätes werden die Steuerparameter beispielsweise an eine Steuerungseinheit des medizinischen Bildgebungsgerätes weitergegeben, welche Steuerungseinheit zumindest ein Bauteil gemäß den Steuerparametern ansteuert. Falls ein Steuerparameter keine physikalische Größe ist, kann beispielsweise die Steuerungseinheit den Steuerparameter vorzugsweise in zumindest eine physikalische Größe übertragen.

Das erfindungsgemäße Verfahren wird typischerweise nicht für ein konkretes Untersuchungsobjekt ausgeführt. Das erfindungsgemäße Verfahren wird typischerweise ausgeführt, ohne dass ein Patient untersucht wird und/oder ohne dass ein Patient derart positioniert ist, dass eine Untersuchung mit dem medizinischen Bildgebungsgerät ausführbar wäre. Es erfolgt bei Ausführung des erfindungsgemäßen Verfahrens typischerweise keine Ansteuerung des medizinischen Bildgebungsgerätes. Insbesondere werden bei Ausführung des erfindungsgemäßen Verfahrens typischerweise keine Bilddaten von einem Patienten im Rahmen einer klinischen Untersuchung erzeugt.

Zunächst wird zumindest ein Systemparameter des medizinischen Bildgebungsgerätes ermittelt. Der Systemparameter charakterisiert vorzugsweise zumindest eine Eigenschaft des medizinischen Bildgebungssystems. Der Systemparameter kann beispielsweise Information zu einem vom medizinischen Bildgebungsgerät umfassten Bauteil umfassen. Beispielsweise können aus einem Systemparameter eine Option und/oder eine Limitation für einen Steuerungsparameter und somit für ein Protokoll extrahiert werden. Die Ermittlung des zumindest einen Systemparameters des medizinischen Bildgebungsgerätes erfolgt vorzugsweise automatisch anhand eines Algorithmus.

Ein Benutzer des medizinischen Bildgebungsgerätes ist typischerweise eine Person, die das medizinische Bildgebungsgerät während der medizinischen Bildgebung überwacht und/oder die Protokolle für eine Untersuchung eines Patienten auswählt. Ein Benutzer kann ein Radiologe sein, der die mit dem medizinischen Bildgebungsgerät erzeugten Bilddaten befundet. Ein Benutzer kann ein Techniker sein, der bei der Installation des medizinischen Bildgebungsgeräts dessen Funktionalität überprüft und auf die Bedürfnisse einer Person abstimmt, welche Person das medizinische Bildgebungsgerät bedient.

Der Benutzer gibt eine Anforderung vor, wobei diese Anforderung vorzugsweise die Aufnahme von medizinischen Bilddaten betrifft. Es wird zumindest ein Protokoll erstellt, welches die Anforderung berücksichtigt und/oder vorzugsweise einhält. Der Benutzer gibt erfindungsgemäß zwei oder mehrere Anforderungen vor, die bei der Erstellung von zumindest einem Protokoll der Favoritenmenge berücksichtigt werden. Der Benutzer priorisiert bei Vorgabe der zumindest zwei Anforderungen die Anforderungen. Die Priorisierung der Anforderungen werden bei Ermittlung einer Favoritenmenge berücksichtigt. Wird das medizinische Bildgebungsgerät gemäß diesem Protokoll angesteuert, so werden die Anforderungen bei der Aufnahme der Bilddaten typischerweise eingehalten. Eine Anforderung kann auch die Bilddaten betreffen, beispielsweise die Auflösung der Bilddaten. Die Anforderung ist nicht auf ein individuelles Untersuchungsobjekt oder speziell auf einen Patienten abgestimmt. Vorzugsweise gibt die Anforderung eine generelle Eigenschaft bei einer Untersuchung an. Die Anforderung gibt vorzugsweise eine generelle Richtlinie an für jede Messung mit dem medizinischen Bildgebungsgerät, bei der diese Anforderung gelten soll.

Basierend auf dem zumindest einen ermittelten Systemparameter und den zumindest zwei vorgegebenen Anforderungen kann eine Favoritenmenge ermittelt werden. Die Favoritenmenge umfasst typischerweise zumindest ein Protokoll. Vorzugsweise werden mehrere Anforderungen vom Benutzer vorgegeben, die mehrere Protokolle betreffen. Die Favoritenmenge umfasst vorzugsweise mehrere Protokolle, sodass alle vom Benutzer vorgegebenen Anforderungen erfüllt werden können. Die von der Favoritenmenge umfassten Protokolle können vorzugsweise für mehrere, voneinander verschiedene, klinische Fragestellungen und/oder Untersuchungen eingesetzt werden. Die Protokolle der Favoritenmenge stehen vorzugsweise für alle Messungen mit dem medizinischen Bildgebungsgerät zur Verfügung und können von einem Benutzer des medizinischen Bildgebungsgerätes bei einer konkreten Untersuchung ausgewählt werden. Der Benutzer kann bei einer Untersuchung Protokolle abhängig vom Untersuchungsobjekt und/oder der klinischen Fragestellung aus der Favoritenmenge auswählen. Vorzugsweis entsprechen die in der Favoritenmenge enthaltenen Protokolle den Anforderungen des Benutzers, sodass vorzugsweise keine oder geringe Anpassungen der Protokolle aus der Favoritenmenge an die tatsächliche Untersuchungssituation erforderlich sind.

Der Vorteil des erfindungsgemäßen Verfahrens liegt darin, dass die Favoritenmenge Protokolle enthält, die den Anforderungen des Benutzers entsprechen. Der Benutzer kann bei Nutzung des medizinischen Bildgebungsgerätes auf Protokolle der Favoritenmenge zugreifen, wobei die Favoritenmenge gemäß den Anforderungen des Benutzers gestaltet wurde. Gemäß dem erfindungsgemäßen Verfahren kann die Favoritenmenge vom Benutzer nach den individuellen Anforderungen des Benutzers angepasst werden. Das Verfahren ist einfach und/oder intuitiv von einem Benutzer ausführbar. Auch der Detailgrad der zu berücksichtigenden Anforderungen kann vom Benutzer bestimmt werden.

Das erfindungsgemäße Verfahren kann von dem Benutzer ausgeführt werden, welcher Benutzer das medizinische Bildgebungsgerät während der medizinischen Bildgebung überwacht und/oder die Protokolle für eine Untersuchung eines Patienten auswählt, also beispielsweise von einer medizinisch-technischen Assistentin, und/oder von einem Radiologen. Zur Durchführung des erfindungsgemäßen Verfahrens ist vorzugsweise kein Techniker erforderlich, der beispielsweise bei der Installation des medizinischen Bildgebungsgeräts dessen Funktionalität überprüft und auf die Bedürfnisse eines Radiologen abstimmt. Dadurch kann die Favoritenmenge von einem Benutzer erzeugt werden, welcher Benutzer das Magnetresonanzgerät alltäglich benutzt. Durch die Vorgabe der gewünschten Anforderungen kann die Favoritenmenge von dem Benutzer, der das Magnetresonanzgerät alltäglich benutzt, nach dessen Wünschen angepasst werden. Dadurch können Probleme, die bei der Kommunikation zwischen einem Techniker und einem Benutzer, der das Magnetresonanzgerät alltäglich benutzt, vermieden werden. Außerdem ist die Anwesenheit eines Technikers für die Erzeugung einer Favoritenmenge nicht erforderlich, was eine Zeitersparnis und/oder Kostenersparnis bewirken kann.

Das erfindungsgemäße Verfahren kann beispielsweise ausgeführt werden, wenn sich eine Anforderung von einem Benutzer an das medizinische Bildgebungsgerät ändert. Die kann beispielsweise zutreffen, wenn das medizinische Bildgebungsgerät von einem neuen Benutzer verwendet wird. Ist das medizinische Bildgebungsgerät beispielsweise in einer Radiologischen Praxis angeordnet, so kann eine Ausführung des erfindungsgemäßen Verfahrens bei Eintritt eines neuen Radiologen in die Praxis ausgeführt werden. Eine Anforderung von einem Benutzer an das medizinische Bildgebungsgerät kann sich ändern, wenn der Benutzer beispielsweise an einer Schulung für ein derartiges medizinisches Bildgebungsgerät teilgenommen hat und/oder sich Vergütungsrichtlinien für Untersuchungen mit dem medizinischen Bildgebungsgerät ändern.

Das erfindungsgemäße Verfahren kann beispielsweise ausgeführt werden, wenn sich ein Systemparameter des medizinischen Bildgebungsgerätes verändert. Dadurch kann die Favoritenmenge auch bei kleinen Veränderungen des medizinischen Bildgebungsgerätes leicht aktualisiert werden, sodass die Favoritenmenge die neuesten Systemparameter des medizinischen Bildgebungsgerätes ausschöpfen kann. Ist das medizinische Bildgebungsgerät beispielsweise ein Magnetresonanzgerät, so ändert sich beispielsweise bei Anschaffung einer anderen lokalen Empfangsspule ein Systemparameter. Die Protokolle der Favoritenmenge, die mit dieser lokalen Empfangsspule kompatibel sind, können beispielsweise gemäß dem erfindungsgemäßen Verfahren von einem Benutzer, der das Magnetresonanzgerät alltäglich benutzt, angepasst und somit typischerweise verbessert werden. Die Favoritenmenge kann so an den neuen Systemparameter angepasst werden. Bei Vorliegen einer aktuellen, an die individuellen Wünsche des Benutzers angepassten Favoritenmenge können bei Untersuchungen die individuellen und gewünschten Protokolle schnell und zuverlässig angewendet werden. So können mit einem medizinischen Bildgebungsgerät reproduzierbare Ergebnisse und/oder Bilddaten erzeugt werden und/oder eine kontinuierliche Qualität der Bilddaten gewährleistet werden.

In einer vorteilhaften Ausführungsform des Verfahrens wird das Verfahren nur bei einer Neukonfiguration des medizinischen Bildgebungsgerätes ausgeführt. Bei einer Neukonfiguration des medizinischen Bildgebungsgerätes wird typischerweise ein Systemparameter geändert und/oder das medizinische Bildgebungsgerät wird erstmalig bei einem Benutzer angeordnet und/oder neu installiert und/oder in Betrieb genommen. Das Verfahren wird demnach vorzugsweise dann ausgeführt, wenn kein Patient untersucht wird, sondern vielmehr bei einer Inbetriebnahme des medizinischen Bildgebungsgerätes. Das Verfahren wird beispielsweise nach einer Installation und/oder Änderung der Software und/oder Änderung eines Bauteils des medizinischen Bildgebungsgerätes ausgeführt.

Der Vorteil dieser Ausführungsform besteht darin, dass zur Erzeugung der Favoritenmenge kein Techniker erforderlich ist. Der Benutzer kann gemäß der Ausführungsform des Verfahrens sicherstellen, dass die von ihm erzeugte Favoritenmenge die aktuellen Systemparameter berücksichtigt. Dies bewirkt einen verbesserte Individualisierung der Favoritenmenge und eine Kostenersparnis. Insbesondere bei einer Neukonfiguration des medizinischen Bildgebungsgerätes ist eine Anpassung der Protokolle an die Anforderungen des Benutzers erforderlich. Kann diese Anpassung durch den Benutzer in Abwesenheit eines Technikers vorgenommen werden, so können bei jeder Neukonfiguration Kosten und/oder Zeit gespart werden.

In einer vorteilhaften Ausführungsform des Verfahrens wird bei Bestimmung der Favoritenmenge eine Teilmenge aus einer Grundmenge, welche Grundmenge mehrere Protokolle zur Ansteuerung des medizinischen Bildgebungsgerätes umfasst, ausgewählt. Die Grundmenge umfasst vorzugsweise eine Vielzahl an Protokollen, welche abhängig von zumindest einer Anforderung und/oder abhängig von zumindest einem Systemparameter sind. Die Grundmenge ist vorzugsweise auf einer Speichereinheit hinterlegt, sodass die Grundmenge der Ausführungsform des Verfahrens bereitgestellt werden kann. Bei Ausführung des Verfahrens kann bei Bestimmung der Favoritenmenge basierend auf dem zumindest einem Systemparameter und basierend auf der zumindest einen bereitgestellten Anforderung zumindest ein Protokoll der Grundmenge selektiert werden. Die Grundmenge kann beispielsweise von Spezialisten hinsichtlich verschiedener Anforderungen generiert werden. Ein Spezialist kann beispielsweise eine Person sein, die im Umfeld der Entwicklung des medizinischen Bildgebungsgerätes arbeitet. Ein Spezialist kann beispielsweise eine Person sein, die die Anwendung des medizinischen Bildgebungsgerätes, insbesondere als Mediziner und/oder gemeinsam mit einem Mediziner, anpasst, insbesondere optimiert. Die Grundmenge kann beispielsweise bei Neukonfiguration des medizinischen Bildgebungsgerätes bereitgestellt werden, beispielsweise auf einer Speichereinheit. Die Speichereinheit kann vom medizinischen Bildgebungsgerät umfasst werden.

Der Vorteil dieser Ausführungsform besteht darin, dass mit der Grundmenge Protokolle bereitgestellt werden können, welche vorab beispielsweise von einem Spezialisten entwickelt und/oder getestet wurden. Für die von der Grundmenge umfassten Protokolle kann demnach eine definierte Qualität gewährleistet werden. Werden bei Erzeugung der Favoritenmenge Protokolle aus der Grundmenge selektiert, die die zumindest eine gegebene Anforderung und/oder den zumindest einen Systemparameter erfüllen, so kann gleichzeitig eine Individualisierung erzielt werden und eine definierte Qualität der Protokolle gewährleistet sein. Es sind keine Kenntnisse über eine Entwicklung eines Protokolls erforderlich, um die Favoritenmenge entsprechend gewünschten Anforderungen zu generieren. Der Benutzer kann demnach auch mit nur geringen oder keinen Kenntnissen über die Funktionsweise des medizinischen Bildgebungsgerätes Protokolle nach seinen Anforderungen selektieren und eine Favoritenmenge erzeugen.

In einer vorteilhaften Ausführungsform des Verfahrens umfasst der Systemparameter einen der folgenden Parameter:
- Softwareversion des medizinischen Bildgebungsgerätes,
- Vorhandensein und/oder Ausführungsform eines speziellen Bauteiles des medizinischen Bildgebungsgerätes.

Die Softwareversion kann eine Version einer Software sein, welche Software beispielsweise zu einer Ansteuerung des medizinischen Bildgebungsgerätes und/oder zu einer Auswertung der mit dem medizinischen Bildgebungsgerät aufgenommenen Daten und/oder zu einer Erzeugung der Bilddaten geeignet ist. Zur Verwendung der Softwareversion und/oder eines Teiles der Softwareversion kann der Erwerb einer Lizenz an der Softwareversion erforderlich sein. Der Systemparameter kann auch das Vorhandensein einer Lizenz an der Softwareversion sein. Ein medizinisches Bildgebungsgerät umfasst typischerweise mehrere Bauteile, welche zur Erzeugung von Bilddaten erforderlich sind. Abhängig von der Art des medizinischen Bildgebungsgerätes kann dies beispielsweise ein Röntgendetektor, ein Ultraschallkopf oder eine Spule zum Senden und/oder Empfang von elektromagnetischen Wellen sein. Ebenso kann ein Computer ein vom medizinischen Bildgebungsgerät umfasstes Bauteil sein. Das spezielle Bauteil betrifft vorzugsweise insbesondere die Ausführbarkeit zumindest eines von der Favoritenmenge umfassten Protokolls.

Insbesondere bei Magnetresonanzgeräten stellen die vom Magnetresonanzgerät umfassten Sende- und/oder Empfangsspulen eine Limitation für eine Ausführbarkeit von Protokollen dar.

Die Sende- und/oder Empfangsspulen können typischerweise unabhängig vom Magnetresonanzgerät erworben werden. Insbesondere lokale Sende- und/oder Empfangsspulen können vorzugsweise intuitiv verwendet werden, sodass eine Einweisung durch einen Techniker typischerweise nicht erfolgt. Für eine Ausnutzung der Funktionalität einer Sende- und/oder Empfangsspule ist typischerweise eine Aktualisierung der Favoritenmenge, insbesondere der Protokolle, welche Protokolle dazu ausgebildet sind, dieses Bauteil bei Ansteuerung des Magnetresonanzgerätes zu gebrauchen, besonders vorteilhaft.

Der Vorteil dieser Ausführungsform des Verfahrens liegt darin, dass die Favoritenmenge einfach an eine aktuelle Ausgestaltungsform des medizinischen Bildgebungsgerätes angepasst werden kann. Dadurch kann sichergestellt werden, dass der Benutzer die aktuelle Ausgestaltungsform des medizinischen Bildgebungsgerätes bestmöglich ausnutzt. Es ist denkbar, dass das medizinische Bildgebungsgerät eine Änderung eines Systemparameters erkennt und einen Benutzer auffordert, das erfindungsgemäße Verfahren auszuführen, insbesondere dazu auffordert, die Favoritenmenge gemäß dem geänderten Systemparameter anzupassen. Insbesondere bei Änderungen des medizinischen Bildgebungsgerätes, die keine Anwesenheit eines Technikers erfordern, wie beispielsweise der Kauf einer Spule und/oder eine fernüberwachte und/oder ferngesteuerte Aktualisierung der Software, kann die Favoritenmenge nach der Änderung einfach an die individuellen Bedürfnisse des Benutzers angepasst werden. Die Änderung der Favoritenmenge kann vom Benutzer selbst ausgeführt werden, was im Vergleich zu einer komplexen Ausführung, die die Anwesenheit eines Technikers erfordert, eine Zeit- und/oder Kostenersparnis bewirkt.

Erfindungsgemäß umfasst die Anforderung zumindest eine der folgenden Eigenschaften:
- Qualität der zu erzeugenden Bilddaten,
- maximale und/oder minimale Dauer eines Protokolls,
- maximale und/oder minimale Dauer für eine Menge an Protokollen, welche für eine Untersuchung eines Untersuchungsobjektes erforderlich sind,
- einen Untersuchungsbereich,
- eine klinische Fragestellung,
- Erfordernis eines Kontrastmittels,
- Automatisierungsgrad,
- Möglichkeit einer individuellen Nachbearbeitung eines Protokolls.

Der Benutzer des medizinischen Bildgebungsgerätes kann vorzugsweise zumindest eine der genannten Eigenschaften als Anforderung für die Bestimmung einer Favoritenmenge vorgeben.

Eine Qualität der zu erzeugenden Bilddaten bezieht sich auf eine Eigenschaft der Bilddaten, welche mit dem medizinischen Bildgebungsgerät erzeugbar sind. Eine Qualität der Bilddaten kann beispielsweise durch eine Auflösung und/oder durch eine Unschärfe und/oder durch einen Kontrast und/oder durch eine Anfälligkeit der Bilddaten für Bewegungen des Untersuchungsobjektes bestimmt sein. Dabei bezieht sich die Qualität der Bilddaten auf die Bilddaten, die durch Ansteuerung des medizinischen Bildgebungsgerätes gemäß einem Protokoll, das die zumindest eine vorgegebene Anforderung erfüllt, erzeugbar sind. Die genannte Qualität ist vorzugsweise eine Eigenschaft, die unabhängig von dem Untersuchungsobjekt gilt. Der Vorteil dieser Anforderung liegt darin, dass der Benutzer eine minimal erforderliche Qualität vorgeben kann, die insbesondere für jedes Untersuchungsobjekt eingehalten werden kann. Dies kann einen definierten Qualitätsstandard für die Bilddaten sicherstellen.

Eine weitere vorgebbare Anforderung kann eine maximale und/oder minimale Dauer eines Protokolls sein, insbesondere der maximale und/oder der minimale Zeitraum, der zum Ausspielen des Protokolls erforderlich ist. Die maximale Dauer kann dabei ein oberes Limit für die Dauer angeben, wobei das Protokoll für die Favoritenmenge vorzugsweise derart gewählt wird, dass es kürzer als die angegebene maximale Dauer dauert. Je kürzer ein Protokoll dauert, desto geringer ist typischerweise die Wahrscheinlichkeit, dass beim Ausspielen des Protokolls, also bei der Ansteuerung des medizinischen Bildgebungsgerätes gemäß diesem Protokoll, Artefakte aufgrund einer Bewegung des Untersuchungsobjektes auftreten. Dies kann die Qualität der Bilddaten erhöhen. Zusätzlich kann die Vorgabe einer maximalen Dauer eines Protokolls die Dauer begrenzen, welche für die Untersuchung eines Untersuchungsobjektes erforderlich ist. Dies kann eine Zeitersparnis bewirken. Die Vorgabe einer minimalen Dauer kann hingegen die Qualität der Bilddaten erhöhen, da bei einer längeren Messdauer typischerweise eine höhere Qualität der Bilddaten erzielt werden kann.

Des Weiteren kann der Benutzer eine maximale und/oder minimale Dauer für eine Menge an Protokollen angeben, welche für eine Untersuchung eines Untersuchungsobjektes erforderlich sind. Für die Untersuchung eines Untersuchungsobjektes können, abhängig von der Art des medizinischen Bildgebungsgerätes, mehrere Bilddaten für einen Teilbereich des Untersuchungsobjektes erforderlich sein. Die mehreren Bilddaten werden dabei typischerweise durch die Ansteuerung des medizinischen Bildgebungsgerätes mittels verschiedenen Protokollen erzeugt. Diese verschiedenen Protokolle können als Menge an Protokollen, welche für eine Untersuchung eines Untersuchungsobjektes erforderlich sind, bezeichnet werden. Kann der Benutzer die maximale und/oder minimale Dauer für eine Menge an Protokollen vorgeben, so kann die Dauer für eine vollständige Untersuchung eines Patienten vorhergesagt werden, was eine zuverlässige Planbarkeit garantieren kann. Darüber hinaus kann die Vorgabe einer maximalen Dauer eine Zeit- und/oder Kostenersparnis bewirken.

Eine weitere Anforderung kann ein Untersuchungsbereich sein. Ein Untersuchungsbereich ist typischerweise eine Region des Untersuchungsobjektes, beispielsweise eine Körperteil oder Teil eines Gliedmaßes, von dem mittels dem medizinischen Bildgebungsgerät bei einer Untersuchung Bilddaten erzeugt werden. Beispiele für einen Untersuchungsbereich kann der Kopf, Abdomen, Knie oder Hüfte sein. Die Untersuchungen verschiedener Untersuchungsbereiche erfordern typischerweise verschiedene Protokolle. So ist bei einer Kopfuntersuchung die Größe des Untersuchungsbereiches und/oder die Auflösung der Bilddaten typischerweise anders als bei einer Untersuchung eines Abdomen. Diese Parameter in einem Protokoll der Favoritenmenge berücksichtigt werden.

Der Untersuchungsbereich kann genauer spezifiziert werden, insbesondere indem eine klinische Fragestellung angegeben werden kann. Beispielsweise sind bei Untersuchungen des Herzens mittels eines Magnetresonanzgerätes bei einer möglichen Ischämie andere Kontraste vorteilhaft als bei der Kontrolle einer Herzklappenfunktion. Die klinische Fragestellung kann auch unabhängig vom Untersuchungsbereich als Anforderung durch den Benutzer vorgegeben werden.

Insbesondere bei Untersuchungen mittels Magnetresonanzgeräten, Computertomographen und/oder Positron-Emissions-Tomographen (PET) ist die Verabreichung von Kontrastmitteln an das Untersuchungsobjekt vor und/oder bei Untersuchungen möglich. Der Benutzer kann beispielsweise als Anforderung vorgeben, ob dem Untersuchungsobjekt ein Kontrastmittel verabreicht werden soll. Zusätzlich kann beispielsweise der Zeitpunkt der Verabreichung vorgegeben werden. Typischerweise ist die Verabreichung des Kontrastmittels abhängig von dem Untersuchungsbereich und/oder der klinischen Fragestellung. Das Erfordernis eines Kontrastmittels wird vorzugsweise in Abhängigkeit des Untersuchungsbereiches und/oder der klinischen Fragestellung angegeben. Der Benutzer kann demnach individuell angeben, bei welchen Untersuchungen ein Kontrastmittel eingesetzt werden soll.

Als weitere Anforderung kann beispielsweise der Automatisierungsgrad angegeben werden. Der Automatisierungsgrad bezieht sich vorzugsweise auf einen Ablauf einer Untersuchung, bei welcher Untersuchung Bilddaten von einem Untersuchungsobjekt erzeugt werden. Beispielsweise kann bei einem hohen Automatisierungsgrad die Favoritenmenge derart ausgestaltet sein, dass bei einer Untersuchung nach Positionierung des Untersuchungsobjektes vorzugsweise maximal fünf Interaktionen, besonders bevorzugt maximal drei Interaktionen zwischen einem Benutzer des medizinischen Bildgebungsgerätes und dem medizinischen Bildgebungsgerät erforderlich sind, um alle von dem Untersuchungsobjekt erforderlichen Bilddaten zu erzeugen. Durch die Angabe eines gewünschten Automatisierungsgrades bei der Bestimmung der Favoritenmenge kann die Qualifikation eines Benutzers, welcher Benutzer Bilddaten von einem Untersuchungsobjekt basierend auf der Favoritenmenge erzeugt, berücksichtigt werden. Ebenso kann als Anforderung die Möglichkeit für eine individuelle Nachbearbeitung zumindest eines in der Favoritenmenge enthaltenen Protokolls und/oder der Grad der Möglichkeit für eine individuelle Nachbearbeitung angegeben werden. Die in der Favoritenmenge entsprechen vorzugsweise weitgehend den Anforderungen des Benutzers. Bei der Anwendung eines Protokolls bei einer konkreten Untersuchung eines Untersuchungsobjektes kann es vorteilhaft sein, weitere Steuerparameter an das Untersuchungsobjekt anzupassen, sodass das verwendete Protokoll von dem in der Favoritenmenge enthaltenen Protokoll abweichen kann. Die Möglichkeit für eine individuelle Anpassung eines Protokolls ermöglicht also eine Optimierung eines Protokolls für eine spezielle Untersuchung. Dadurch kann gegebenenfalls die Qualität der Bilddaten verbessert werden. Für die Einhaltung einer einheitlichen Qualität bei verschiedenen Untersuchungen und/oder bei geringer Qualifikation eines Benutzers kann es vorteilhaft sein, keine oder eine sehr geringe individuelle Nachbearbeitung der von der Favoritenmenge umfassten Protokolle zu ermöglichen. Dadurch kann bei geringer Qualifikation eines Benutzers eine minimale Qualität sichergestellt werden. Einem Benutzer mit hoher Qualifikation kann für die Erzeugung von Bilddaten mehrere Freiheitsgrade ermöglicht werden, sodass diese die Qualität der Bilddaten hinsichtlich des Untersuchungsobjektes und/oder der klinischen Fragestellung optimierbar ist. Der Vorteil der Ausführungsform besteht darin, dass die Qualifikation des zumindest einen Benutzers berücksichtigt werden kann.

Weitere, dem Fachmann als sinnvoll erscheinende Anforderungen können in einer alternativen Ausgestaltung der Erfindung jederzeit verwendet werden, ohne den Schutzbereich der Erfindung zu verlassen.

Die genannten möglichen Anforderungen ermöglichen die Berücksichtigung verschiedener individueller Ansprüche bei der Erzeugung der Favoritenmenge, wobei der Detailgrad der Anforderungen individuell bestimmt werden kann. Die gemäß dieser Ausführungsform des erfindungsgemäßen Verfahrens erzeugte Favoritenmenge kann als Grundlage für eine Messung eines Untersuchungsobjektes mittels dem medizinischen Bildgebungsgeräts verwendet werden: Die Favoritenmenge umfasst individuell auf den Benutzer abgestimmte Protokolle, die bei einer Untersuchung einfach ausgewählt werden können. Der Benutzer kann, wie gegebenenfalls bei der Erzeugung der Favoritenmenge angegeben, gegebenenfalls weitere Änderungen der Protokolle vornehmen oder kann bei einem hohen Automatisierungsgrad mit wenigen Interaktionen mit dem medizinischen Bildgebungsgerät die Untersuchung ausführen. Insgesamt wird eine auf der Favoritenmenge basierende Untersuchung typischerweise weniger fehleranfällig.

In einer vorteilhaften Ausführungsform des Verfahrens erfolgt die Vorgabe der Anforderung über eine Eingabeaufforderung an den Benutzer. Eine Eingabeaufforderung kann beispielsweise eine Anzeige auf einem Monitor sein, die den Benutzer eine Frage stellt und/oder mehrere Optionen zur Auswahl stellt. Der Benutzer kann die Frage beantworten und/oder eine Option auswählen. Diese Option kann eine erste Anforderung an die Favoritenmenge sein und/oder eine weitere Eingabeaufforderung induzieren, wobei bei der weiteren Eingabeaufforderung eine weitere Anforderung eingegebenen werden kann und die weitere Anforderung die erste Anforderung genauer spezifizieren kann. Eine derartige Ausgestaltung des Verfahrens ist für den Benutzer besonders leicht verständlich und intuitiv zu bedienen. Dadurch kann der Benutzer angeleitet werden, sodass sämtliche für die Favoritenmenge relevanten Anforderungen über typischerweise mehrere Eingabeaufforderungen akquiriert werden können. Gemäß dieser Ausführungsform können die Anforderungen des Benutzers eindeutig verarbeitet werden.

In einer vorteilhaften Ausführungsform des Verfahrens sind mehrere Anforderungen iterativ vorgebbar, wobei eine erste der mehreren Anforderungen durch eine zweite der mehreren Anforderungen genauer spezifiziert wird. Der Benutzer kann gemäß dieser Ausführungsform verschiedene Anforderungen an die Favoritenmenge stellen, wobei eine erste Anforderung einen ersten Detailgrad aufweist und eine zweite Anforderung einen zweiten Detailgrad aufweist. Eine Anforderung mit dem ersten Detailgrad kann durch eine Anforderung mit dem zweiten Detailgrad genauer spezifiziert werden. Nach der ersten und/oder der zweiten Anforderung kann der Benutzer vorzugsweise bestimmen, ob er die Anforderung durch eine weitere Anforderung, beispielsweise eines dritten Detailgrades, detaillierter bestimmen möchte oder eine weitere Anforderung des ersten und/oder zweiten Detailgrades auswählen möchte. Das Verfahren ist vorzugsweise derart ausgestaltet, dass die Vorgabe der Anforderungen der verschiedenen Detailgrade intuitiv möglich ist und beispielsweise einer bekannten Baumstruktur folgt. Gemäß dieser Ausführungsform kann sichergestellt werden, dass alle für den Benutzer interessanten Anforderungen vorgegeben werden können und/oder der Detailgrad vom Benutzer individuell, gegebenenfalls abhängig von der ersten Anforderung, gestaltet werden kann. Dies ermöglicht eine sehr flexible Vorgabe verschiedener Anforderungen und/oder einen hohen Individualisierungsgrad.

Erfindungsgemäß wird bei Nichterfüllung von zumindest einer Anforderung bei Bestimmung der Favoritenmenge dem Benutzer ein Protokoll zur Auswahl vorgeschlagen, welches die zumindest eine Anforderung weitgehend erfüllt.

Die Bestimmung der Favoritenmenge erfolgt unter Berücksichtigung des zumindest einen Systemparameters und der zumindest zwei Anforderungen. Die von der Favoritenmenge umfassten Protokolle sind vorzugsweise mit dem zumindest einem Systemparameter kompatibel und erfüllen vorzugsweise alle vorgegebenen Anforderungen. Abhängig von der Wahl der zumindest zwei Anforderungen und/oder des Systemparameters können Widersprüche auftreten, sodass kein Protokoll vorhanden ist und/oder erzeugt werden kann, das ausspielbar ist und die zumindest eine vorgegebene Anforderung, insbesondere alle vorgegebenen Anforderungen, erfüllt. In diesem Fall kann ein Protokoll erzeugt und/oder selektiert werden, das die zumindest zwei Anforderungen weitgehend erfüllt. Eine Anforderung ist weitgehend erfüllt, wenn die Abweichung des Protokolls von der Anforderung minimal ist.

Wurden zwei oder mehrere Anforderungen vorgegebenen, so ist eine Abweichung weitgehend erfüllt, wenn die Gesamtheit der Abweichungen des Protokolls von den Anforderungen minimiert wird. Insbesondere bei zwei gegensätzlichen Anforderungen, wie beispielsweise eine hohe Auflösung und eine geringe Messdauer, kann ein Protokoll typischerweise nicht beide Anforderungen gleichzeitig erfüllen. Die Anforderungen werden bei deren Vorgabe durch den Benutzer priorisiert und/oder gewichtet, was bei Erzeugung der Favoritenmenge berücksichtigt wird. Die Priorisierung und/oder Wichtung kann berücksichtigt werden, wenn ein Protokoll zur Auswahl vorgeschlagen wird, welches die zumindest eine Anforderung weitgehend erfüllt.

Das Protokoll, welches die zumindest eine Anforderung weitgehend erfüllt, kann dem Benutzer vorgeschlagen werden. Vorzugsweise wird dem Benutzer die Abweichung im Vergleich mit seiner Anforderung dargestellt und/oder die Möglichkeit gegeben, dieses Protokoll auszuwählen und/oder zumindest eine Anforderung zu ändern. Der Vorteil dieser Ausführungsform ist, dass der Benutzer bei einer Nichterfüllung einer Anforderung informiert wird und ihm eine bestmögliche Lösung vorgeschlagen wird.

In einer vorteilhaften Ausführungsform des Verfahrens werden die Protokolle der Favoritenmenge derart klassifiziert, dass die für eine Untersuchung eines Untersuchungsobjektes erforderlichen Protokolle eine gleiche Klassifizierung aufweisen. Eine Klassifizierung der Protokolle kann vom Benutzer ausgeführt werden. Eine Klassifizierung der Protokolle kann von einem Spezialisten ausgeführt werden. Bei der Klassifizierung wird zumindest einem von der Favoritenmenge umfassten Protokoll zumindest eine Klasse zugewiesen. Eine Klasse kann beispielsweise eine Untersuchungsregion angeben und/oder eine klinische Fragestellung. Eine Klasse charakterisiert vorzugsweise eine Untersuchung, bei der das Protokoll verwendet wird. Einem Protokoll können mehrere Klassen zugeordnet werden.

Ist das medizinische Bildgebungsgerät beispielsweise ein Magnetresonanzgerät, so können bei Ansteuerung des Magnetresonanzgerätes mit einem ersten Protokoll beispielsweise Bilddaten mit einem T2-Kontrast in einer transversalen Orientierung erzeugt werden. Bei Ansteuerung des Magnetresonanzgerätes mit einem zweiten Protokoll können beispielsweise Bilddaten mit einem Tl-Kontrast in einer sagittalen Orientierung erzeugt werden. Das erste Protokoll und das zweite Protokoll werden vorzugsweise für eine Untersuchung von Knien verwendet. Das erste Protokoll und das zweite Protokoll werden demnach beispielsweise mit der Klasse "Knie" klassifiziert. Das erste Protokoll wird beispielsweise bei Untersuchungen des Meniskus, das zweite Protokoll wird beispielsweise bei Untersuchungen des Knorpels verwendet. Das erste Protokoll kann demnach mit der Klasse "Meniskus", das zweite Protokoll kann demnach mit der Klasse "Knorpel" klassifiziert werden.

Der Vorteil dieses Verfahrens liegt darin, dass eine klassifizierte Favoritenmenge einfach durchsucht werden kann. Ein Benutzer kann aus der Favoritenmenge intuitiv für eine Untersuchung relevante Protokolle selektieren. Dies verkürzt demnach die Vorbereitungsdauer für eine Untersuchung und/oder die Fehler, die bei Untersuchungen auftreten können.

In einer vorteilhaften Ausführungsform des Verfahrens wird bei der Bestimmung der Favoritenmenge ein Favoritenprofil berücksichtigt und ein selbstlernender Algorithmus aktualisiert das Favoritenprofil basierend auf der Favoritenmenge. Die Favoritenmenge kann basierend auf zumindest einem Systemparameter, auf zumindest einer Anforderung und einem Favoritenprofil bestimmt werden. Das Favoritenprofil kann eine Information über den Benutzer und/oder dessen Nutzung von dem und/oder einem weiteren medizinischen Bildgebungsgerät umfassen. Das Favoritenprofil kann eine Information über einen weiteren Benutzer und/oder dessen Nutzung von dem und/oder einem weiteren medizinischen Bildgebungsgerät umfassen. Das Favoritenprofil umfasst vorzugsweise Informationen über zumindest eine Anforderung von zumindest einem Benutzer. Das Favoritenprofil ist vorzugsweise auf einer Speichereinheit hinterlegt, sodass die Grundmenge der Ausführungsform des Verfahrens bereitgestellt werden kann.

Hat der Benutzer eine Favoritenmenge erzeugt, so kann das Favoritenprofil beispielsweise derart aktualisiert werden, dass Informationen über den Benutzer und/oder dessen zumindest eine Anforderung und/oder einen Systemparameter in das Favoritenprofil aufgenommen, insbesondere gespeichert, werden. Der selbstlernende Algorithmus kann diese Schritte vorzugweise automatisch ausführen. Der selbstlernende Algorithmus kann vorzugsweise auf mehreren medizinischen Bildgebungsgeräten vernetzt ausgeführt werden. Der selbstlernende Algorithmus ist vorzugsweise derart ausgestaltet, dass er basierend auf dem Favoritenprofil, der Anforderung und des Systemparameters eine Favoritenmenge erzeugen kann, die den Wünschen des Benutzers noch besser entspricht.

In einer vorteilhaften Ausführungsform des Verfahrens wird bei der Bestimmung der Favoritenmenge ein Favoritenprofil berücksichtigt, basierend auf der Favoritenmenge führt ein Benutzer eine weitere Individualisierung der Favoritenmenge aus, und ein selbstlernender Algorithmus aktualisiert das Favoritenprofil basierend auf der individualisierten Favoritenmenge.

Nach Erzeugung der Favoritenmenge kann die Favoritenmenge und/oder von der Favoritenmenge umfasste Protokolle vorzugsweise von dem individualisiert, also beispielsweise nachbearbeitet werden. Insbesondere können die von der Favoritenmenge umfassten Protokolle bei der Individualisierung genauer an die Bedürfnisse des Benutzers angepasst werden. Bei der Individualisierung wird vorzugsweise zumindest ein Steuerparameter der Protokolle verändert. Die Individualisierung erfolgt typischerweise unabhängig von einem Untersuchungsobjekt. Die individualisierte Favoritenmenge wird vorzugsweise von dem selbstlernenden Algorithmus bei der Aktualisierung des Favoritenprofils berücksichtigt. Wird eine weitere Favoritenmenge unter Berücksichtigung des Favoritenprofils umfassend die individualisierte Favoritenmenge erzeugt, so ist typischerweise die Art und/oder das Ausmaß der Individualisierung durch den Benutzer bekannt und kann bei der weiteren Favoritenmenge berücksichtigt werden. Die weitere Favoritenmenge entspricht demnach vorzugsweise möglichst genau den Anforderungen und/oder auch den nicht in Form von Anforderungen geäußerten Wünschen des Benutzers.

In einer vorteilhaften Ausführungsform des Verfahrens umfasst das Favoritenprofil zwei Favoritenmengen, wobei eine erste der zwei Favoritenmengen einem ersten medizinischen Bildgebungsgerät und eine zweite der zwei Favoritenmengen einem zweiten medizinischen Bildgebungsgerät zugeordnet ist. Das Favoritenprofil umfasst demnach vorzugsweise Favoritenmengen, die mit zwei voneinander verschiedenen medizinischen Bildgebungsgeräten erzeugt wurden. Die beiden medizinischen Bildgebungsgeräte sind vorzugsweise medizinische Bildgebungsgeräte der gleichen Modalität, beispielweise beides Magnetresonanzgeräte. Die medizinischen Bildgebungsgeräte können auch eine unterschiedliche Modalität aufweisen, wobei insbesondere bestimmte Anforderungen des Benutzers, wie beispielsweise eine maximale Dauer für ein Protokoll, auf die verschiedenen medizinischen Bildgebungsgeräte übertragen werden können. Das Favoritenprofil umfasst vorzugsweise Favoritenmengen und/oder individualisierte Favoritenmengen und/oder Systemparameter und/oder Anforderungen von einem oder zumindest zwei Benutzern und/oder Informationen über die Benutzer. Basierend darauf kann der selbstlernende Algorithmus vorzugsweise bei Berücksichtigung des Favoritenprofils bei der Erzeugung einer weiteren Favoritenmenge Wünsche des Benutzers besonders gut erkennen und/oder berücksichtigen.

Des Weiteren geht die Erfindung aus von einem medizinischen Bildgebungsgerät mit einer Steuerungseinheit, die eine Bestimmungseinheit umfasst. Die Bestimmungseinheit ist dazu ausgebildet, ein erfindungsgemäßes Verfahren zu einer Erzeugung einer Favoritenmenge umfassend mehrere Protokolle, wobei jedes Protokoll der mehreren Protokolle Steuerparameter zur Ansteuerung des medizinischen Bildgebungsgerätes umfasst, auszuführen.

Dafür weist die Bestimmungseinheit typischerweise einen Eingang, eine Prozessoreinheit und einen Ausgang auf. Über den Eingang können der Bestimmungseinheit eine Grundmenge mehrerer Protokolle und/oder ein Favoritenprofil bereitgestellt werden. Weitere, im Verfahren benötigte Funktionen, Algorithmen oder Parameter können der Bestimmungseinheit über den Eingang bereitgestellt werden. Die Favoritenmenge und/oder weitere Ergebnisse einer Ausführungsform des erfindungsgemäßen Verfahrens können über den Ausgang bereitgestellt werden. Die Bestimmungseinheit kann in das medizinische Bildgebungsgerät integriert sein. Die Bestimmungseinheit kann auch separat von dem medizinischen Bildgebungsgerät installiert sein. Die Bestimmungseinheit kann mit dem medizinischen Bildgebungsgerät verbunden sein.

Ausführungsformen des erfindungsgemäßen medizinischen Bildgebungsgerätes sind analog zu den Ausführungsformen des erfindungsgemäßen Verfahrens ausgebildet. Das medizinische Bildgebungsgerät kann weitere Steuerungskomponenten aufweisen, welche zum Ausführen eines erfindungsgemäßen Verfahrens nötig und/oder vorteilhaft sind. Auch kann das medizinische Bildgebungsgerät dazu ausgebildet sein, Steuerungssignale zu senden und/oder Steuerungssignale zu empfangen und/oder zu verarbeiten, um ein erfindungsgemäßes Verfahren auszuführen. Vorzugsweise ist die Bestimmungseinheit Teil der Steuerungseinheit des erfindungsgemäßen medizinischen Bildgebungsgeräts. Auf einer Speichereinheit der Bestimmungseinheit können Computerprogramme und weitere Software gespeichert sein, mittels derer die Prozessoreinheit der Bestimmungseinheit einen Verfahrensablauf eines erfindungsgemäßen Verfahrens automatisch steuert und/oder ausführt.

Ein erfindungsgemäßes Computerprogrammprodukt ist direkt in einen Speicher einer programmierbaren Bestimmungseinheit ladbar und weist Programmcode-Mittel auf, um ein erfindungsgemäßes Verfahren auszuführen, wenn das Computerprogrammprodukt in der Bestimmungseinheit ausgeführt wird. Dadurch kann das erfindungsgemäße Verfahren schnell, identisch wiederholbar und robust ausgeführt werden. Das Computerprogrammprodukt ist so konfiguriert, dass es mittels der Bestimmungseinheit die erfindungsgemäßen Verfahrensschritte ausführen kann. Die Bestimmungseinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor einer lokalen Bestimmungseinheit geladen werden kann, der mit dem medizinischen Bildgebungsgerät direkt verbunden oder als Teil des medizinischen Bildgebungsgeräts ausgebildet sein kann. Weiterhin können Steuerinformationen des Computerprogrammprodukts auf einem elektronisch lesbarer Datenträger gespeichert sein. Die Steuerinformationen des elektronisch lesbaren Datenträgers können derart ausgestaltet sein, dass sie bei Verwendung des Datenträgers in einer Bestimmungseinheit eines medizinischen Bildgebungsgeräts ein erfindungsgemäßes Verfahren durchführen. Beispiele für elektronisch lesbare Datenträger sind eine DVD, ein Magnetband oder einen USB-Stick, auf welchem elektronisch lesbare Steuerinformationen, insbesondere Software, gespeichert ist. Wenn diese Steuerinformationen (Software) von dem Datenträger gelesen und in eine Steuerungseinheit und/oder Bestimmungseinheit eines medizinischen Bildgebungsgeräts gespeichert werden, können alle erfindungsgemäßen Ausführungsformen der vorab beschriebenen Verfahren durchgeführt werden.

Des Weiteren geht die Erfindung aus von einem elektronisch lesbaren Datenträger, auf dem ein Programm hinterlegt ist, das zu einer Ausführung eines Verfahrens zu einem Ausspielen einer MR-Steuerungssequenz, vorgesehen ist.

Die Vorteile des erfindungsgemäßen medizinischen Bildgebungsgeräts, des erfindungsgemäßen Computerprogrammprodukts und des erfindungsgemäßen elektronisch lesbaren Datenträgers entsprechen im Wesentlichen den Vorteilen des erfindungsgemäßen Verfahrens zu einer Erzeugung einer Favoritenmenge umfassend mehrere Protokolle, wobei jedes Protokoll der mehreren Protokolle Steuerparameter zur Ansteuerung des medizinischen Bildgebungsgerätes umfasst, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: ein erfindungsgemäßes medizinisches Bildgebungsgerät in einer schematischen Darstellung,
- Fig. 2: ein Ablaufdiagramm einer ersten Ausführungsform eines erfindungsgemäßen Verfahrens, und
- Fig. 3: ein Schema für eine Vorgabe einer Anforderung durch einen Benutzer.

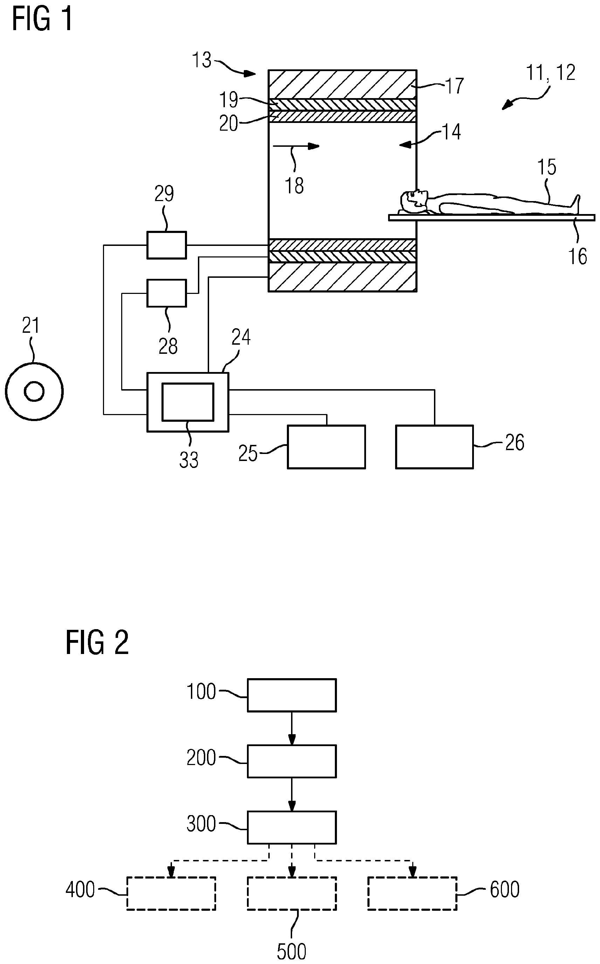

Figur 1 zeigt ein Magnetresonanzgerät 11 als Beispiel für ein erfindungsgemäßes medizinisches Bildgebungsgerät 12 zur Ausführung eines erfindungsgemäßen Verfahrens in einer schematischen Darstellung. Grundsätzlich ist die Ausgestaltung des medizinischen Bildgebungsgerätes 12 nicht auf ein Magnetresonanzgerät 11 eingeschränkt, sondern kann auch von weiteren, dem Fachmann als sinnvoll erscheinenden medizinischen Bildgebungsgeräten 12, wie beispielsweise Computertomographen, PET, Ultraschallgeräten, usw. gebildet sein.

Das Magnetresonanzgerät 11 umfasst eine von einer Magneteinheit 13 gebildeten Detektoreinheit mit einem Hauptmagneten 17 zu einem Erzeugen eines starken und insbesondere konstanten Hauptmagnetfelds 18. Zudem weist das Magnetresonanzgerät 11 einen zylinderförmigen Patientenaufnahmebereich 14 zu einer Aufnahme eines Patienten 15 auf, wobei der Patientenaufnahmebereich 14 in einer Umfangsrichtung von der Magneteinheit 13 zylinderförmig umschlossen ist. Der Patient 15 kann mittels einer Patientenlagerungsvorrichtung 16 des Magnetresonanzgeräts 11 in den Patientenaufnahmebereich 14 geschoben werden. Die Patientenlagerungsvorrichtung 16 weist hierzu einen Patiententisch auf, der bewegbar innerhalb des Magnetresonanzgeräts 11 angeordnet ist.

Die Magneteinheit 13 weist weiterhin eine Gradientenspuleneinheit 19 auf, die für eine Ortskodierung während einer Bildgebung verwendet werden. Die Gradientenspuleneinheit 19 wird mittels einer Gradientensteuereinheit 28 angesteuert. Des Weiteren weist die Magneteinheit 13 eine Hochfrequenzantenneneinheit 20, welche im gezeigten Fall als fest in das Magnetresonanzgerät 11 integrierte Körperspule ausgebildet ist, und eine Hochfrequenzantennensteuereinheit 29 zu einer Anregung einer Polarisation, die sich in dem von dem Hauptmagneten 17 erzeugten Hauptmagnetfeld 18 einstellt, auf. Die Hochfrequenzantenneneinheit 20 wird von der Hochfrequenzantennensteuereinheit 29 angesteuert und strahlt hochfrequente Hochfrequenz-Pulse in einen Untersuchungsraum, der im Wesentlichen von dem Patientenaufnahmebereich 14 gebildet ist, ein.

Zu einer Steuerung des Hauptmagneten 17, der Gradientensteuereinheit 28 und der Hochfrequenzantennensteuereinheit 29 weist das Magnetresonanzgerät 11 eine Steuerungseinheit 24 auf. Die Steuerungseinheit 24 steuert zentral das Magnetresonanzgerät 11, wie beispielsweise das Durchführen von MR-Steuerungssequenzen. Zudem umfasst die Steuerungseinheit 24 eine nicht näher dargestellte Rekonstruktionseinheit zu einer Rekonstruktion von medizinischen Bilddaten, die während der Magnetresonanzuntersuchung erfasst werden. Das Magnetresonanzgerät 11 weist eine Anzeigeeinheit 25 auf. Steuerinformationen wie beispielsweise Steuerungsparameter, sowie rekonstruierte Bilddaten können auf einer Anzeigeeinheit 25, beispielsweise auf zumindest einem Monitor, des Magnetresonanzgeräts 11 für einen Benutzer angezeigt werden. Zudem weist das Magnetresonanzgerät 11 eine Eingabeeinheit 26 auf, mittels derer Informationen und/oder Bildgebungsparameter während eines Messvorgangs von einem Benutzer eingegeben werden können. Die Steuerungseinheit 24 kann die Gradientensteuereinheit 28 und/oder Hochfrequenzantennensteuereinheit 29 und/oder die Anzeigeeinheit 25 und/oder die Eingabeeinheit 26 umfassen.

Die Steuerungseinheit 24 umfasst weiterhin eine Bestimmungseinheit 33. Die Bestimmungseinheit 33 ist zudem zu einer Ausführung eines Verfahrens zu einer Erzeugung einer Favoritenmenge umfassend mehrere Protokolle ausgelegt. Hierzu weist die Bestimmungseinheit 33 Computerprogramme und/oder Software auf, die direkt in einem nicht näher dargestellten Speichereinheit der Bestimmungseinheit 33 ladbar sind, mit Programmmitteln, um ein Verfahren zu einer Erzeugung einer Favoritenmenge umfassend mehrere Protokolle auszuführen, wenn die Computerprogramme und/oder Software in der Bestimmungseinheit 33 ausgeführt werden. Die Bestimmungseinheit 33 weist hierzu einen nicht näher dargestellten Prozessor auf, der zu einer Ausführung der Computerprogramme und/oder Software ausgelegt ist. Alternativ hierzu können die Computerprogramme und/oder Software auch auf einem getrennt von der Steuerungseinheit 24 und/oder Bestimmungseinheit 33 ausgebildeten elektronisch lesbaren Datenträger 21 gespeichert sein, wobei ein Datenzugriff von der Bestimmungseinheit 33 auf den elektronisch lesbaren Datenträger 21 über ein Datennetz erfolgen kann.

Das dargestellte Magnetresonanzgerät 11 kann selbstverständlich weitere Komponenten umfassen, die Magnetresonanzgeräte 11 gewöhnlich aufweisen. Eine allgemeine Funktionsweise eines Magnetresonanzgeräts 11 ist zudem dem Fachmann bekannt, so dass auf eine detaillierte Beschreibung der weiteren Komponenten verzichtet wird. Das Magnetresonanzgerät 11 ist somit zusammen mit der Bestimmungseinheit 33 zur Ausführung eines erfindungsgemäßen Verfahrens ausgelegt.

Ein Verfahren zu einer Erzeugung einer Favoritenmenge umfassend mehrere Protokolle kann auch in Form eines Computerprogrammprodukts vorliegen, das das Verfahren auf die Bestimmungseinheit 33 implementiert, wenn es auf der Bestimmungseinheit 33 ausgeführt wird. Ebenso kann ein elektronisch lesbarer Datenträger 21 mit darauf gespeicherten elektronisch lesbaren Steuerinformationen vorliegen, welche zumindest ein solches eben beschriebenes Computerprogrammprodukt umfassen und derart ausgestaltet sind, dass sie bei Verwendung des Datenträgers 21 in einer Bestimmungseinheit 33 eines Magnetresonanzgeräts 11 das beschriebene Verfahren durchführen.

Figur 2 zeigt ein Ablaufdiagramm einer ersten Ausführungsform eines erfindungsgemäßen Verfahrens zu einer Erzeugung einer Favoritenmenge umfassend mehrere Protokolle, wobei jedes Protokoll der mehreren Protokolle Steuerparameter zur Ansteuerung eines medizinischen Bildgebungsgerätes umfasst. Das dargestellte Verfahren wird vorzugsweise nur bei einer Neukonfiguration des medizinischen Bildgebungsgerätes 12 ausgeführt.

Das Verfahren beginnt vorzugsweise mit Verfahrensschritt 100, der Ermittlung eines Systemparameters des medizinischen Bildgebungsgerätes 12. Es können dabei auch zwei oder mehr Systemparameter ermittelt werden. Ein Systemparameter kann beispielsweise eine Softwareversion des medizinischen Bildgebungsgerätes 12 und/oder das Vorhandensein und/oder eine Ausführungsform eines speziellen Bauteiles des medizinischen Bildgebungsgerätes umfassen.

In Verfahrensschritt 200 erfolgt die Vorgabe zumindest einer Anforderung durch einen Benutzer des medizinischen Bildgebungsgerätes 12, bevor in Verfahrensschritt 300 vorzugsweise mittels der Bestimmungseinheit 33 die Favoritenmenge unter Berücksichtigung des zumindest einen Systemparameters und der Anforderung bestimmt wird. Die Anforderung kann zumindest eine der folgenden Eigenschaften umfassen:
- Qualität der zu erzeugenden Bilddaten,
- maximale und/oder minimale Dauer eines Protokolls,
- maximale und/oder minimale Dauer für eine Menge an Protokollen, welche für eine Untersuchung eines Untersuchungsobjektes erforderlich sind,
- einen Untersuchungsbereich,
- eine klinische Fragestellung,
- Erfordernis eines Kontrastmittels,
- Automatisierungsgrad,
- Möglichkeit einer individuellen Nachbearbeitung eines Protokolls.

Bei Bestimmung der Favoritenmenge in Verfahrensschritt 300 wird vorzugsweise eine Teilmenge aus einer Grundmenge ausgewählt, welche Grundmenge mehrere Protokolle zur Ansteuerung des medizinischen Bildgebungsgerätes 12 umfasst. Die Grundmenge wird dafür vorzugsweise der Steuerungseinheit 24, insbesondere der Bestimmungseinheit 33, bereitgestellt.

Kann zumindest eine Anforderung der in Verfahrensschritt 200 vorgegebenen zumindest einen Anforderung in Verfahrensschritt 300 bei Bestimmung der Favoritenmenge nicht erfüllt werden, so kann dem Benutzer optional in Verfahrensschritt 400 ein Protokoll zur Auswahl vorgeschlagen werden, welches die zumindest eine Anforderung weitgehend erfüllt.

Optional können in Verfahrensschritt 500 die Protokolle der Favoritenmenge derart klassifiziert werden, dass die für eine Untersuchung eines Untersuchungsobjektes erforderlichen Protokolle eine gleiche Klassifizierung aufweisen.

Optional kann in Verfahrensschritt 300 bei der Bestimmung der Favoritenmenge ein Favoritenprofil berücksichtigt werden. In Verfahrensschritt 600 kann optional ein Benutzer basierend auf der Favoritenmenge eine weitere Individualisierung der Favoritenmenge des medizinischen Bildgebungsgerätes ausführen und ein selbstlernender Algorithmus kann das Favoritenprofil basierend auf der individualisierten Favoritenmenge aktualisieren. Alternativ und/oder zusätzlich kann der selbstlernende Algorithmus das Favoritenprofil basierend auf der in Verfahrensschritt 300 bestimmten Favoritenmenge aktualisieren. Das Favoritenprofil kann zwei Favoritenmengen umfassen, wobei eine erste der zwei Favoritenmengen einem ersten Magnetresonanzgerät und eine zweite der zwei Favoritenmengen einem zweiten Magnetresonanzgerät zugeordnet ist.

Die Verfahrensschritte 400, 500 und 600 können optional und unabhängig voneinander ausgeführt werden. Zumindest zwei der Verfahrensschritte 400, 500 und 600 können zumindest teilweise gleichzeitig ausgeführt werden. Zumindest zwei der Verfahrensschritte 400, 500 und 600 können nacheinander ausgeführt werden.

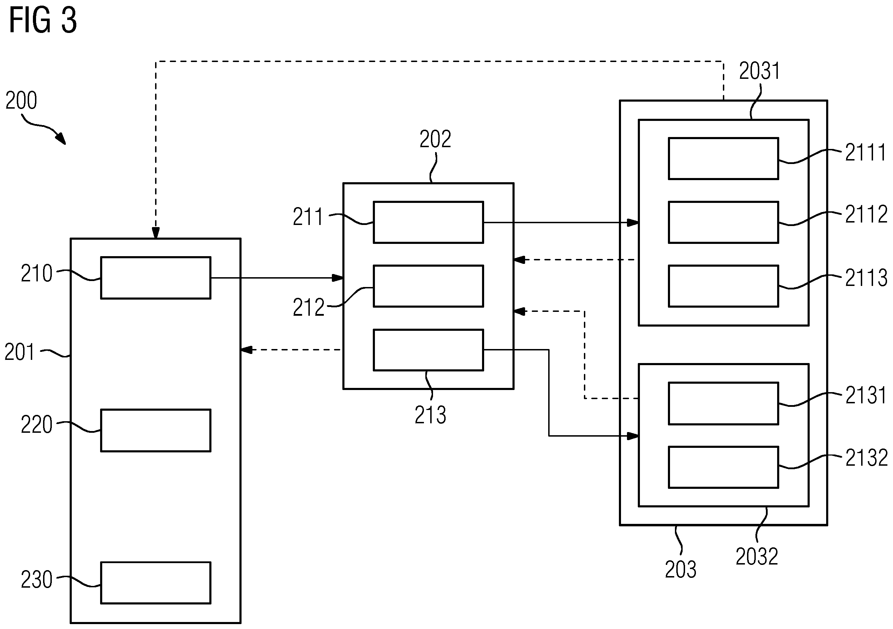

Figur 3 zeigt ein Schema für eine Vorgabe einer Anforderung durch einen Benutzer und gibt ein detailliertes Beispiel für eine Ausführungsform von Verfahrensschritt 200. Die Vorgabe der Anforderung erfolgt typischerweise über eine Eingabeaufforderung an den Benutzer. Hierbei sind mehrere Anforderungen 201, 202, 203 iterativ vorgebbar, wobei eine erste 201 der mehreren Anforderungen 201, 202, 203 durch eine zweite 202 und gegebenenfalls durch eine dritte 203 der mehreren Anforderungen 201, 202, 203 genauer spezifiziert werden kann. Beispielsweise kann die erste Anforderung 201 eine Untersuchungsregion, die zweite Anforderung 202 eine klinische Fragestellung für diese Untersuchungsregion und die dritte Anforderung 203 eine erwünschte maximale Untersuchungsdauer sein.

Beispielsweise kann der Benutzer zunächst dazu aufgefordert werden, eine erste Anforderung 201 anzugeben, indem der Benutzer eine der vorgegebenen Untersuchungsregionen 210, 220, 230 auswählen soll. Wählt der Benutzer als erste Anforderung die Untersuchungsregion 210, beispielsweise das Knie, aus, so kann der Benutzer als nächstes dazu aufgefordert werden, als zweite Anforderung 202 eine klinische Fragestellung einzugeben. Beispielsweise können hier der Knorpel 212, das Kreuzband 212 und der Meniskus 213 zur Untersuchung aufgeführt sein. Abhängig von der Wahl kann der Benutzer optional dazu aufgefordert werden, die Untersuchung durch die Angabe weiterer Anforderungen 203, 2031, 2032 weiter zu spezifizieren. Hier können beispielsweise abhängig von der zweiten Anforderung 202 dem Benutzer unterschiedliche Anforderungen 2031, 2032 vorgeschlagen werden. So kann bei der Wahl des Knorpels 212 der zweiten Anforderung 202 beispielsweise ausgewählt werden, ob die Untersuchung mit Kontrastmittel 2121 ausgeführt werden soll, wie lange die Untersuchung des Knorpels maximal dauern darf 2122 und ob die Untersuchung des Knorpels eine Interaktion des Benutzers mit dem Magnetresonanzgerät erfordern soll (Automatisierungsgrad) 2123. Würde als zweite Anforderung 202 der Meniskus 213 ausgewählt werden, so könnte als dritte Anforderung 203, 2032 beispielsweise die maximale Dauer der Untersuchung 2131 und die Verwendung eines Kontrastmittels 2132 ausgewählt werden.

Nach der Wahl der zweiten Anforderung 202 und/oder der Wahl der dritten Anforderung 203 kann eine neue erste Anforderung 201 und/oder eine neue zweite Anforderung 202 ausgewählt werden. Die Interaktion des Benutzers wird dabei vorzugsweise derart gesteuert, dass alle für ihn relevanten Anforderungen abgefragt werden, sodass der Benutzer die Favoritenmenge möglichst weitgehend individualisieren kann.

## Patentansprüche

1. Verfahren, ausgeführt durch ein medizinisches Bildgebungsgerät (12) mit einer Steuerungseinheit (24), die eine Bestimmungseinheit (33) umfasst, zu einer Erzeugung einer Favoritenmenge umfassend mehrere Protokolle, wobei jedes Protokoll der mehreren Protokolle Steuerparameter zur Ansteuerung des medizinischen Bildgebungsgerätes (12) umfasst, umfassend die folgenden Verfahrensschritte:
- Ermittlung eines Systemparameters des medizinischen Bildgebungsgerätes (12),
- Vorgabe von zumindest zwei Anforderungen betreffend eine Aufnahme von medizinischen Bilddaten unabhängig von einem individuellen Untersuchungsobjekt (15) durch einen Benutzer des medizinischen Bildgebungsgerätes (12),
- Bestimmung der Favoritenmenge durch Erstellung zumindest eines Protokolls unter Berücksichtigung des Systemparameters und der zumindest zwei Anforderungen mittels der Bestimmungseinheit,
- Bereitstellung der Favoritenmenge über einen Ausgang,
wobei bei Nichterfüllung von zumindest einer der zumindest zwei Anforderungen bei Bestimmung der Favoritenmenge dem Benutzer ein Protokoll zur Auswahl vorgeschlagen wird, welches die Gesamtheit der Abweichungen des Protokolls von den zumindest zwei Anforderungen minimiert, wobei die zumindest zwei Anforderungen bei deren Vorgabe durch den Benutzer priorisiert und/oder gewichtet werden, was bei Bestimmung der Favoritenmenge berücksichtigt wird, wobei die zumindest zwei Anforderungen zumindest eine der folgenden Eigenschaften umfassen:
- Qualität der zu erzeugenden Bilddaten,
- maximale und/oder minimale Dauer eines Protokolls,
- maximale und/oder minimale Dauer für eine Menge an Protokollen, welche für eine Untersuchung eines Untersuchungsobjektes (15) erforderlich sind,
- einen Untersuchungsbereich,
- eine klinische Fragestellung,
- Erfordernis eines Kontrastmittels,
- Automatisierungsgrad,
- Möglichkeit einer individuellen Nachbearbeitung eines Protokolls.

2. Verfahren nach Anspruch 1,
wobei das Verfahren nur bei einer Neukonfiguration des medizinischen Bildgebungsgerätes (12) ausgeführt wird.

3. Verfahren nach einem der vorangehenden Ansprüche,
wobei bei Bestimmung der Favoritenmenge eine Teilmenge aus einer Grundmenge, welche Grundmenge mehrere Protokolle zur Ansteuerung des medizinischen Bildgebungsgerätes (12) umfasst, ausgewählt wird.

4. Verfahren nach einem der vorangehenden Ansprüche,
wobei der Systemparameter einen der folgenden Parameter umfasst:
- Softwareversion des medizinischen Bildgebungsgerätes (12),
- Vorhandensein und/oder Ausführungsform eines speziellen Bauteiles des medizinischen Bildgebungsgerätes (12).

5. Verfahren nach einem der vorangehenden Ansprüche,
wobei die Vorgabe der zumindest zwei Anforderungen über eine Eingabeaufforderung an den Benutzer erfolgt.

6. Verfahren nach einem der vorangehenden Ansprüche,
wobei mehrere Anforderungen iterativ vorgebbar sind, wobei eine erste der mehreren Anforderungen durch eine zweite der mehreren Anforderungen spezifiziert wird.

7. Verfahren nach einem der vorangehenden Ansprüche,
wobei die Protokolle der Favoritenmenge derart klassifiziert werden, dass die für eine Untersuchung eines Untersuchungsobjektes (15) erforderlichen Protokolle eine gleiche Klassifizierung aufweisen.

8. Verfahren nach einem der vorangehenden Ansprüche,
wobei bei der Bestimmung der Favoritenmenge ein Favoritenprofil, umfassend Informationen über zumindest eine Anforderung von zumindest einem Benutzer, berücksichtigt wird und ein selbstlernender Algorithmus das Favoritenprofil basierend auf der Favoritenmenge aktualisiert.

9. Verfahren nach einem der vorangehenden Ansprüche,
wobei bei der Bestimmung der Favoritenmenge ein Favoritenprofil, umfassend Informationen über zumindest eine Anforderung von zumindest einem Benutzer, berücksichtigt wird,
basierend auf der Favoritenmenge ein Benutzer eine weitere Individualisierung der Favoritenmenge ausführt,
und ein selbstlernender Algorithmus das Favoritenprofil basierend auf der individualisierten Favoritenmenge aktualisiert.

10. Verfahren nach Anspruch 8 oder 9,
wobei das Favoritenprofil zwei Favoritenmengen umfasst, wobei eine erste der zwei Favoritenmengen einem ersten Magnetresonanzgerät und eine zweite der zwei Favoritenmengen einem zweiten Magnetresonanzgerät zugeordnet ist.

11. Medizinisches Bildgebungsgerät (12) mit einer Steuerungseinheit, die eine Bestimmungseinheit umfasst, welche zu einer Ausführung eines Verfahrens nach einem der vorangehenden Ansprüche zu einer Erzeugung einer Favoritenmenge umfassend mehrere Protokolle ausgelegt ist.

12. Computerprogrammprodukt, welches ein Programm umfasst und direkt in einen Speicher einer programmierbaren Bestimmungseinheit, welche Bestimmungseinheit (33) von einer Steuerungseinheit (24) eines medizinischen Bildgebungsgerätes (12) umfasst wird, ladbar ist, mit Programmmitteln, um ein Verfahren zu einer Erzeugung einer Favoritenmenge umfassend mehrere Protokolle nach einem der Ansprüche 1 bis 10 auszuführen, wenn das Programm in der Bestimmungseinheit ausgeführt wird.

13. Elektronisch lesbarer Datenträger (21), auf dem ein Programm hinterlegt ist, das derart ausgestaltet ist, dass das Programm bei Verwendung des Datenträgers in einer Bestimmungseinheit, welche Bestimmungseinheit (33) von einer Steuerungseinheit (24) eines medizinischen Bildgebungsgerätes (12) umfasst wird, das Verfahren zu einer Erzeugung einer Favoritenmenge umfassend mehrere Protokolle nach einem der Ansprüche 1 bis 10 durchführt.

## Claims

1. Method, executed by a medical imaging device (12) with a control unit (24), which comprises a definition unit (33), for generating a favourites set comprising a plurality of protocols, wherein each protocol of the plurality of protocols comprises control parameters for controlling the medical imaging device (12), comprising the following method steps:
- determination of a system parameter of the medical imaging device (12),
- specification of at least two requirements by a user of the medical imaging device (12) relating to a recording of medical image data independently of an individual examination object (15),
- definition of the favourites set by creating at least one protocol, taking into account the system parameter and the at least two requirements by means of the definition unit,
- providing the favourites set by way of an output, wherein, if at least one of the at least two requirements is not fulfilled when the favourites set is defined, a protocol, which minimises the totality of the deviations of the protocol from the at least two requirements, is proposed to the user for selection, wherein the at least two requirements are prioritised and/or weighted by the user upon their specification, which is taken into account when defining the favourites set, wherein the at least two requirements comprise at least one of the following characteristics:
- quality of the image data to be generated,
- maximum and/or minimum duration of a protocol,
- maximum and/or minimum duration for a set of protocols which are required for an examination of an examination object (15),
- an examination area,
- a clinical objective,
- need for a contrast medium,
- degree of automation,
- possibility of custom post-processing of a protocol.

2. Method according to claim 1,
wherein the method is executed only upon reconfiguration of the medical imaging device (12).

3. Method according to one of the preceding claims,
wherein, when the favourites set is defined, a subset is selected from a core set, which core set comprises a plurality of protocols for controlling the medical imaging device (12).

4. Method according to one of the preceding claims,
wherein the system parameter comprises one of the following parameters:
- software version of the medical imaging device (12),
- presence and/or embodiment of a specific component of the medical imaging device (12).

5. Method according to one of the preceding claims,
wherein the specification of the at least two requirements is effected via an input request to the user.

6. Method according to one of the preceding claims,
wherein a plurality of requirements are iteratively specifiable, wherein a first of the plurality of requirements is specified by a second of the plurality of requirements.

7. Method according to one of the preceding claims,
wherein the protocols of the favourites set are classified such that the protocols required for an examination of an examination object (15) have an identical classification.

8. Method according to one of the preceding claims,
wherein, when the favourites set is defined, a favourites profile, comprising information relating to at least one requirement by at least one user, is taken into account and a self-learning algorithm updates the favourites profile based on the favourites set.

9. Method according to one of the preceding claims,
wherein, when the favourites set is defined, a favourites profile comprising information relating to at least one requirement by at least one user is taken into account,
based on the favourites set, a user executes a further customisation of the favourites set,
and a self-learning algorithm updates the favourites profile based on the customised favourites set.

10. Method according to claim 8 or 9,
wherein the favourites profile comprises two favourites sets, wherein a first of the two favourites sets is assigned to a first magnetic resonance device and a second of the two favourites sets is assigned to a second magnetic resonance device.

11. Medical imaging device (12) with a control unit which comprises a definition unit which is designed to execute a method according to one of the preceding claims for generating a favourites set comprising a plurality of protocols.

12. Computer program product which comprises a program and can be loaded directly into a memory of a programmable definition unit, which definition unit (33) is included in a control unit (24) of a medical imaging device (12), with program means for executing a method according to one of claims 1 to 10 for generating a favourites set comprising a plurality of protocols when the program is executed in the definition unit.

13. Electronically readable data medium (21), on which a program is filed which is designed such that, when the data medium is used in a definition unit, which definition unit (33) is included in a control unit (24) of a medical imaging device (12), the program implements the method according to one of claims 1 to 10 for generating a favourites set comprising a plurality of protocols.

## Revendications

1. Procédé exécuté par un appareil (12) d'imagerie médicale comprenant une unité (24) de commande, qui comprend une unité (33) de détermination pour une production d'un ensemble de favoris comprenant plusieurs protocoles, dans lequel chaque protocole parmi les plusieurs protocoles comprend des paramètres de commande pour la commande de l'appareil (12) d'imagerie médicale, comprenant les stades de procédé suivants :
- détermination d'un paramètre de système de l'appareil (12) d'imagerie médicale,
- prescription par un utilisateur de l'appareil (12) d'imagerie médicale d'au moins deux demandes concernant un enregistrement de données d'images médicales indépendamment d'un objet (15) individuel en examen,
- détermination au moyen de l'unité de détermination d'un ensemble de favoris par l'établissement d'au moins un protocole en tenant compte du paramètre de système et des au moins deux demandes,
- mise à disposition d'un ensemble de favoris par une sortie, dans lequel si au moins l'une des au moins deux demandes n'est pas satisfaite à la détermination d'un ensemble de favoris, on propose à l'utilisateur un protocole de sélection, qui minimise la totalité des écarts du protocole aux au moins deux demandes, dans lequel les au moins deux demandes sont, lors de leur prescription, mises en priorité par l'utilisateur et/ou pondérées, ce que l'on prend en compte à la détermination d'un ensemble de favoris, dans lequel les au moins deux demandes comprennent au moins l'une des propriétés suivantes :
- qualité des données d'image à produire,
- durée maximum et/ou minimum d'un protocole,
- durée maximum et/ou minimum d'un ensemble de protocoles, qui sont nécessaires pour un examen d'un objet (15) à examiner,
- une partie à examiner,
- la pose d'une question clinique,
- la nécessité d'un agent de contraste,
- un degré d'automatisation,
- une possibilité d'un retraitement individuel d'un protocole.

2. Procédé suivant la revendication 1,
dans lequel on n'exécute le procédé que s'il y a une configuration nouvelle de l'appareil (12) d'imagerie médicale.

3. Procédé suivant l'une des revendications précédentes,
dans lequel à la détermination de l'ensemble de favoris on sélectionne un ensemble partiel d'un ensemble de base, lequel ensemble partiel comprend plusieurs protocoles de commande de l'appareil (12) d'imagerie médicale.

4. Procédé suivant l'une des revendications précédentes,
dans lequel le système de paramètre comprend l'un des paramètres suivants :
- une version logicielle de l'appareil (12) d'imagerie médicale,
- présence et/ou forme de réalisation d'un élément spécial de l'appareil (12) d'imagerie médicale.

5. Procédé suivant l'une des revendications précédentes,
dans lequel la prescription des au moins deux demandes s'effectue par une invitation d'entrée à l'utilisateur.

6. Procédé suivant l'une des revendications précédentes,
dans lequel plusieurs demandes peuvent être prescrites itérativement, dans lequel on spécifie une première des plusieurs demandes par une deuxième des plusieurs demandes.

7. Procédé suivant l'une des revendications précédentes,
dans lequel on classe les protocoles de l'ensemble des favoris de manière à ce que les protocoles nécessaires à un examen d'un objet (15) à examiner aient une même classification.

8. Procédé suivant l'une des revendications précédentes,
dans lequel à la détermination de l'ensemble de favoris, on prend en compte un profil de favori comprenant des informations sur au moins une demande d'au moins un utilisateur et un algorithme d'auto-apprentissage met à jour le profil de favori sur la base de l'ensemble de favoris.

9. Procédé suivant l'une des revendications précédentes,
dans lequel à la détermination de l'ensemble de favoris, on prend en compte un profil de favori comprenant de l'information sur au moins une demande d'au moins un utilisateur,
sur la base de l'ensemble de favoris, un utilisateur exécute une autre individualisation de l'ensemble de favoris,
et un algorithme d'auto-apprentissage met à jour le profil de favori sur la base de l'ensemble de favoris individualisés.

10. Procédé suivant l'une des revendications 8 ou 9,
dans lequel le profil de favori comprend deux ensembles de favori, dans lequel un premier des deux ensembles de favori est associé à un premier appareil à résonnance magnétique et un deuxième des deux ensembles de favoris est associé à un deuxième appareil à résonnance magnétique.

11. Appareil (12) d'imagerie médicale comprenant une unité de commande, qui comprend une unité de détermination, qui est conçue pour l'exécution d'un procédé suivant l'une des revendication précédentes pour une production d'un ensemble de favoris comprenant plusieurs protocoles.

12. Produit de programme d'ordinateur, qui comprend un programme, et qui peut être chargé directement dans une mémoire d'une unité programmable de détermination, ladite unité (33) de détermination étant comprise par une unité (24) de commande de l'appareil (12) d'imagerie médicale, comprenant des moyens de programme pour exécuter un procédé de production d'un ensemble de favoris comprenant plusieurs protocoles suivant l'une des revendications 1 à 10 lorsque le programme est réalisé dans l'unité de détermination.

13. Support (21) de données, déchiffrable électroniquement, sur lequel est mis en mémoire un programme qui est conformé de manière à ce que le programme lors de l'utilisation du support de données dans une unité de détermination, laquelle unité (33) de détermination est comprise par une unité (24) de commande de l'appareil (12) d'imagerie médicale, exécute le procédé de production d'un ensemble de favoris comprenant plusieurs protocoles suivant l'une des revendications 1 à 10.
